# EUROPÄISCHE PATENTANMELDUNG

(11) **EP 2 929 851 A1**
(43) Veröffentlichungstag der Anmeldung: **14.10.2015**
(21) Anmeldenummer: 14163794.2
(22) Anmeldetag: 08.04.2014
(51) Int. Cl.: A61B 18/14

(54) **Instrument zur Resektion oberflächlicher Läsionen**

(71) Anmelder: Farin, Günter, 72070 Tübingen (DE)
(72) Erfinder: Farin, Günter, 72070 Tübingen (DE); Grund, Karl, 72072 Tübingen (DE)
(74) Vertreter: Jöstingmeier, Martin

(57) **Zusammenfassung**

Ein HF-chirurgisches Instrument zum HF-chirurgischen Entfernen oberflächlicher pathologischer Gewebeschichten von vitalen biologischen Organen oder anderen vitalen Zielgeweben in Form einer Pinzette, Zange oder Klemme, umfasst zwei an ihrer Oberfläche elektrisch nicht leitfähige Branchen die an ihren distalen Enden voneinander beabstandet und nach oben gekröpft oder abgewinkelt sind und so ein Paar schlittenartige Gleitkufen bilden. Ein HF-chirurgischer Schneiddraht ist zwischen den Gleitkufen angeordnet und mittels eines Kabels mit einer HF-Strom-Quelle verbindbar. Dadurch können verschieden dicke bzw. dünne und/oder verschieden breite bzw. schmale oberflächliche Gewebeschichten in sano und en block von vitalen biologischen Organen oder Gewebestrukturen entfernt werden.

## Beschreibung

### Technisches Gebiet

Gegenstand der Erfindung ist ein HF-chirurgisches Instrument, mit welchem auf Oberflächen von Organen oder Gewebestrukturen vorhandene störende oder gar pathologische Gewebe HF-chirurgisch entfernt werden können.

### Stand der Technik

Auf Organen oder Gewebestrukturen oberflächlich vorhandene störende oder gar pathologische Gewebe können chirurgisch entfernt werden, wenn dies der Kosmetik dient oder wenn diese Gewebe zwar gutartig, für den Patienten aber störend sind, und sie müssen entfernt werden, wenn diese Gewebe bösartig, also Krebs- bzw. Karzinomgewebe sind. Die chirurgische Entfernung oberflächlicher Karzinome, wie sie beispielsweise bei der Peritonealkarzinose an mehreren verschiedenen Organen und / oder Gewebestrukturen mit verschiedener Schichtdicke und verschiedener flächigen Ausbreitung vorkommen können, ist schwierig und eine große Herausforderungen an die Chirurgie bzw. die Chirurgen. Hierfür fehlt ein geeignetes chirurgisch anwendbares Instrument mit welchem verschieden dicke pathologische Gewebeschichten von der Oberfläche von Organen oder Gewebestrukturen mit kontrollierter Schnitttiefe entfernt werden können.

Rein mechanische Schälmesser, wie Gemüseschälmesser sind zum Entfernen bzw. Resezieren pathologischer Gewebeschichten von vitalen biologischen Organen in vivo nicht geeignet, weil zum Schneiden eine mechanische Kraft in Schnittrichtung auf das zu resezierende Gewebe und auf bzw. in die Oberfläche des betreffenden Organs hinein wirkt. Dadurch wird das zu resezierende Gewebe und / oder die Oberfläche des betreffenden Organs elastisch und / oder plastisch verformt und / oder der Kraft ausweichend bewegt. Außerdem können hierbei störende oder gar gefährliche Blutungen verursacht werden können.

Aus der DE 101 38 235 C1 ist ein chirurgisches Schälmesser offenbart, mit einem Haltegriff und einem am Haltgriff fixierten Klingenhalter, welcher eine metallische Klinge hält, die quer zu einer Längsrichtung des Haltegriffs orientiert ist. Der Klingenhalter ist so ausgebildet, dass die Klinge versetzt zu einer Längsebene des Haltegriffs angeordnet ist, wobei die Klinge eine erste Elektrode bildet und beabstandet zur Klinge an dem Klingenhalter eine zweite Elektrode angeordnet ist.

Pathologische Gewebeschichten auf der Oberfläche biologischer Organe können nicht nur verschieden dick, sondern auch verschieden breit sein. Onkologen for-dern, dass pathologische Gewebe, insbesondere Karzinomgewebe und / oder Vorstufen von Karzinomgeweben, in sano, also bis in das angrenzende gesunde Gewebe hinein, entfernt werden, und dies sowohl in der Tiefenausdehnung als auch in der Flächenausdehnung. Ob diese Forderung der Onkologen erfüllt wurde oder nicht, muss durch mindestens eine mikroskopische bzw. pathohistologische Untersuchung des entfernten Gewebes bestätigt werden. Um diese Untersuchung zuverlässig durchführen zu können, fordern die Pathologen außerdem die Entfernung pathologischer Gewebe möglichst en bloc, also in einem Stück, und möglichst ohne mechanische und / oder thermische Artefakte (Beschädigungen).

Dicke pathologische Gewebeschichten durch mehrere Schälschnitte an derselben Stelle nacheinander abzuschälen, wie es beispielsweise beim Schälen von Gemüse mit einem Gemüseschälmesser üblich ist, steht der o.g. Forderung der Pathologen entgegen.

### Darstellung der Erfindung

Der Erfindung liegt die Aufgabe zugrunde, ein Verfahren und ein hierfür geeignetes Instrument zu entwickeln, mit welchem verschieden dicke bzw. dünne und/oder verschieden breite bzw. schmale oberflächliche Gewebeschichten in sano (also bis ins angrenzende gesunde Gewebe hinein) und möglichst en block (also in einem Stück) von vitalen (also durchbluteten) biologischen Organen oder Gewebestrukturen entfernt werden können.

Diese Aufgabe wird durch eine Vorrichtung nach Anspruch 1 gelöst. Vorteilhafte Ausgestaltungen der Erfindung sind in den Unteransprüchen angegeben.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
- Figur 1: zeigt ein Ausführungsbeispiel nach Art einer Pinzette.
- Figur 2: zeigt ein Ausführungsbeispiel nach Art einer Zange oder Klemme.

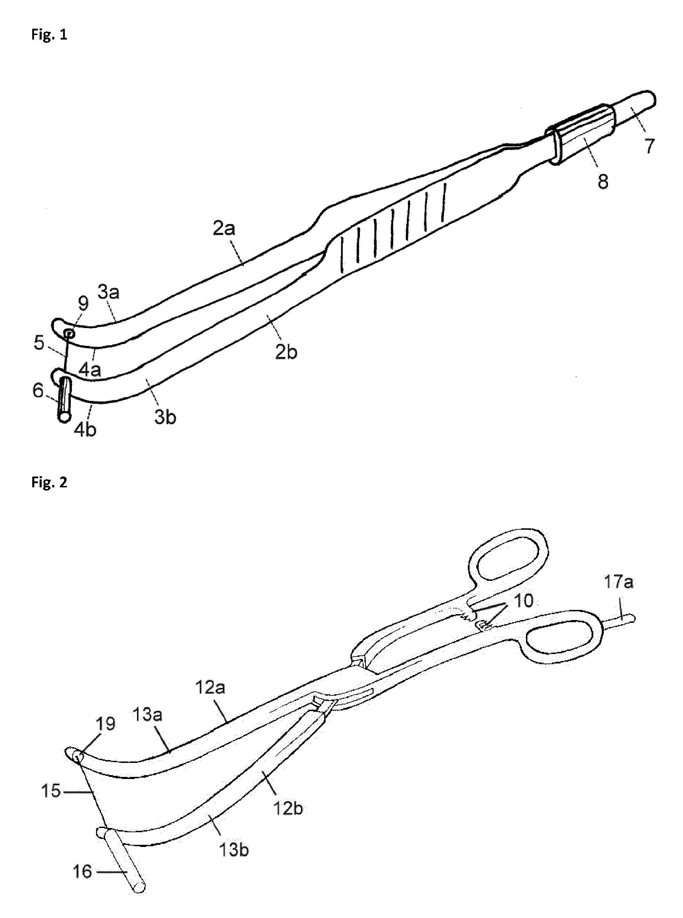

Ein erfindungsgemäßes HF-chirurgisches Resektionsinstrument (1) nach Art einer Pinzette, wie es in Figur 1 dargestellt ist, besteht aus zwei Branchen (2a, 2b) die an ihren proximalen Enden direkt oder mittels eines Verbindungselements (8) mechanisch fest oder beweglich miteinander verbunden und vorzugsweise an ihren distalen Enden (3a, 3b) nach oben gekröpft oder abgewinkelt sind. Die Branchen können mechanisch steif, plastisch oder elastisch verformbar sein. Somit können die Branchen mechanisch fest mit vorgegebenem Abstand oder auch mit variablem Abstand ausgeführt sein. Bevorzugt sind die Branchen Teil einer Pinzette, einer Zange oder Klemme. Zwischen den distalen Enden der vorzugsweise gekröpften oder abgewinkelten Branchen ist ein metallischer Draht (5) als HF-chirurgische Schneidelektrode bzw. HF-chirurgischer Schneiddraht angeordnet, dessen eines Ende am distalen Ende (3a) der Branche (2a) an einer Stelle (9) mechanisch befestigt ist. An der der Stelle (9) des distalen Endes (3a) der Branche 2a gegenüber liegenden Stelle des distalen Endes (3b) befindet sich bevorzugterweise ein Loch (nicht sichtbar), durch welches der Schneiddraht (5) in axialer Richtung frei beweglich hindurch geschoben und/oder gezogen werden kann. Auf diese Weise kann die effektive Länge des HF-chirurgischen Schneiddrahts (5) durch mehr oder weniger weites Zusammendrücken der Branchen (2a, 2b) der jeweiligen Breite des zu Resezierenden Gewebes angepasst werden. Um zu vermeiden, dass der aus dem Loch nach außen herausragende HF-chirurgisch aktivierbare Schneiddraht (5) unbeabsichtigt Verletzungen an Geweben verursacht, ist außerhalb des distalen Endes (3b) der Branche (2b) eine Schutzhülse (6) in axialer Richtung des Drahts (5) vorgesehen, in die der Schneiddraht (5) durch das Loch hindurch eintauchen kann.

Die Leitung des zum HF-chirurgischen Schneiden bzw. Resezieren biologischer Gewebe erforderlichen elektrischen Stroms bzw. HF-Stroms kann durch mindestens eine der beiden Branchen (2a, 2b), hier vorzugsweise durch die Branche (2a), an welcher der Schneiddraht (5) befestigt ist, geleitet werden. Der Anschluss des Instruments an einen HF-Stromgenerator kann durch ein Stromkabel (7) erfolgen, das permanent oder lösbar mit dem Instrument verbunden ist bzw. verbunden werden kann.

Außer dem Schneiddraht (5) ist das Instrument 1 bevorzugt zumindest an den Stellen, die mit dem Chirurgen und/oder Patienten in Kontakt kommen können, elektrisch isoliert. Die Branchen und/oder eine Schutzhülse dieses Instrument können aber auch vollständig aus elektrisch nicht leitfähigem Kunststoff hergestellt werden bzw. aus Kunststoff bestehen, wobei lediglich eine Stromleitung innerhalb mindestens einer Branche eingebettet ist und den Schneiddraht (5) mit dem Kabel (7) verbindet. Alternativ kann ein Stromkabel-Anschluss auch an einer anderen Stelle des Instruments angeordnet sein, beispielsweise direkt an der Stelle (9), wo der HF-chirurgische Schneiddraht (5) mechanisch an der Branche (2a) befestigt ist. Diese Anordnung ist dann vorteilhaft, wenn die Herstellungskosten des Instruments gering sein sollen.

Die unteren Kanten (4a, 4b) der vorzugsweise gekröpften oder angewinkelten Enden (3a, 3b) der Branchen (2a, 2b) dienen bei der Anwendung dieses Instruments als Gleitkufen, die während einer Schnittführung in Schnittrichtung auf der Oberfläche des Organs gleiten.

Durch die bevorzugte Kröpfung bzw. Abwinkelung der distalen Enden (4a, 4b) der Branchen (2a, 2b) kann der Abstand des Schneiddrahts (5) zur Oberfläche des Organs definiert werden. So kann ein oberflächliches pathologisches Gewebe planparallel zu der Oberfläche des Organs HF-chirurgisch abreseziert werden., Durch Anpassung des jeweils geeigneten Anstellwinkels der Branchen zur jeweils betreffenden Oberfläche des Organs kann die Schnittdicke variiert werden. Hierbei ist zu berücksichtigen, dass biologische Gewebe mehr oder weniger elastisch verformbar sind und dass die Gleitkufen durch mehr oder weniger Kraft bzw. Druck gegen die Oberfläche mehr oder wenige tiefe Eindellungen erzeugen, wodurch der vertikale Abstand des Schneiddraht zur Organoberfläche und damit die vertikale Position der Schnittführung zusätzlich variiert werden kann.

In Kombination mit der Möglichkeit, die effektive Länge des Schneiddrahts durch mehr oder weniger weites Zusammendrücken der beiden Branchen der jeweiligen Breite des zu resezierenden Gewebes anzupassen, steht dem Chirurgen nun ein Instrument zur Verfügung, bei dessen Anwendung außerdem durch Wahl geeigneter HF-chirurgischer Parameter am jeweils zur Verfügung stehenden HF-Stromgenerator und/oder durch geeignete Geschwindigkeit der Schnittführung, auch ein schnittsynchroner Verschluss der unvermeidlich durchschnittenen Blutgefäße möglich ist.

Zur weiteren Ausgestaltung der Erfindung kann zwischen den Branchen (2a, 2b) eine Anschlagvorrichtung zur Begrenzung der Weite des Zusammendrückens der Branchen angeordnet sein, wodurch die effektive Länge des Schneiddrahts nicht kürzer als erforderlich eingestellt werden kann, so dass die Länge der Schutzhülse (6) dem entsprechend kurz sein kann.

Zur weiteren Ausgestaltung der Erfindung kann der Durchmesser des Lochs (5) in der Gleitkufe (4b) so dimensioniert sein, dass einerseits der HF-chirurgische Schneiddraht ungehindert hindurch bewegt werden kann und andererseits auf dem Schneiddraht haftendes Gewebe beim Hineinschieben des Schneiddrahts nicht in das Loch mitgenommen sondern vom Schneiddraht abgeschabt wird. Auf diese Weise kann übrigens auf dem Schneiddraht haften bleibendes Gewebe entfernt werden.

Wie in Fig. 2 dargestellt, kann das erfindungsgemäße Instrument beispielsweise auch zangen- oder klemmenartig gestaltet werden. Die einzelnen Komponenten der bzw. an den Branchen entsprechen denjenigen aus der Fig. 1. Das Instrument hat eine erste Branche 12a mit einem ersten distalen Ende 13a und eine zweite gegenüber dieser bewegliche Branche 12b mit einem zweiten distalen Ende 13b. Die beiden Branchen können über die Raste 10 gegeneinander verriegelt werden. Ein metallischer Draht bzw. HF-chirurgischer Schneiddraht 15 ist über einen Stromanschluß 17a mit einem HF-Chirurgiegenerator verbindbar. Der Schneiddraht ist an einem Ende an der Verbindungsstelle 19 mit der Branche 12a verbunden und kann mit dem anderen Ende in die Schutzhülse 16 eintauchen. Vorzugsweise sind auch hier die beiden unteren Kanten, die als Gleitkufen dienen können, vorgesehen.

### Bezugszeichenliste

- 2a: Branche
- 2b: Branche
- 3a: distales Ende der Branche 2a
- 3b: distales Ende der Branche 2b
- 4a: untere Kante, dient als Gleitkufe beim Schneiden
- 4b: untere Kante, dient als Gleitkufe beim Schneiden
- 5: metallischer Draht bzw. HF-chirurgischer Schneiddraht
- 6: Schutzhülse
- 7: Stromkabel, permanent oder lösbar mit dem Instrument verbunden
- 8: Verbindungselement am proximalen Ende der Branchen 2a und 2b
- 9: Verbindungsstelle des Schneiddrahts mit der Branche 2a
- 10: Raste
- 12a: Branche
- 12b: Branche
- 13a: distales Ende der Branche 12a
- 13b: distales Ende der Branche 12b
- 15: metallischer Draht bzw. HF-chirurgischer Schneiddraht
- 16: Schutzhülse
- 17a: Stromanschluß
- 19: Verbindungsstelle des Schneiddrahts mit der Branche 12a

## Patentansprüche

1. HF-chirurgisches Instrument (1) zum HF-chirurgischen Entfernen oberflächlicher pathologischer Gewebeschichten von vitalen biologischen Organen oder anderen vitalen Zielgeweben, umfassend:
- zwei zumindest an ihrer Oberfläche elektrisch nicht leitfähige Branchen (2a, 2b), die an ihrem proximalen Ende direkt oder mittels eines Verbindungselementes (8) mechanisch miteinander verbunden sind und die an ihren distalen Enden (3a, 3b) voneinander beabstandet sind,
- einen HF-chirurgischen Schneiddraht (5) zwischen den Gleitkufen (4a, 4b),
- ein Kabel (7) oder einen Anschluss (7) für ein Kabel zum Leiten eines HF-Stroms von einer HF-Strom-Quelle zum Instrument (1), und
- eine elektrisch leitfähige Verbindung zwischen dem Kabel (7) oder dem Anschluss (7) für ein Kabel zum HF-chirurgischen Schneiddraht (5).

2. HF-chirurgisches Instrument nach Anspruch 1,
**dadurch gekennzeichnet, dass**
die Branchen (2a, 2b) an ihren distalen Enden (3a, 3b) gekröpft oder abgewinkelt sind.

3. HF-chirurgisches Instrument nach Anspruch 2,
**dadurch gekennzeichnet, dass**
die Branchen (2a, 2b) ein Paar schlittenartige Gleitkufen (4a, 4b) bilden.

4. HF-chirurgisches Instrument nach einem der vorhergehenden Ansprüche,
**dadurch gekennzeichnet, dass**
das Instrument nach Art einer Pinzette oder Zange gestaltet ist, so dass der Abstand zwischen den beiden distalen Enden (3a, 3b) der Branchen (2a, 2b) variabel ist, und dass der HF-chirurgische Schneiddraht mechanisch und elektrisch leitfähig fest an einer Stelle (9) am distalen Ende (3a) einer ersten Branche (2a) verbunden ist, und dass am distalen Ende (3b) der anderen Branche (2b)gegenüber dieser Stelle (9) ein Loch vorhanden ist, durch welches der Schneiddraht in seiner axialen Richtung frei beweglich hindurchgeführt ist, so dass der Abstand zwischen den Gleitkufen (4a, 4b) und damit die effektive Länge des HF-chirurgischen Schneiddrahts (5) durch mehr oder weniger weites Zusammendrücken der Branchen (2a, 2b) variierbar ist.

5. HF-chirurgisches Instrument nach Anspruch 4,
**dadurch gekennzeichnet, dass**
an der seitlichen Außenfläche der anderen Branche (2b) eine zumindest an ihrer Oberfläche elektrisch nicht leitfähige Schutzhülse (6) so angeordnet ist, dass ein freies Ende des Schneiddrahts (5) beim Zusammendrücken der Branchen (2a, 2b) durch das Loch in der Gleitkufe (4b) hindurch in die Schutzhülse (6) eintauchen kann.

6. HF-chirurgisches Instrument nach Anspruch 4 oder 5,
**dadurch gekennzeichnet, dass**
zwischen den Branchen (2a,2b) eine Anschlagvorrichtung zur Begrenzung der Weite des Zusammendrückens der Branchen bis zu einem bestimmten Minimalabstand zwischen den distalen Enden der Gleitkufen (4a, 4b) und damit der effektiv verfügbaren Länge des Schneiddrahts (5) angeordnet ist.

7. HF-chirurgisches Instrument nach einem der Ansprüche 4 bis 6,
**dadurch gekennzeichnet, dass**
das Loch in der Gleitkufe (4b) einerseits einen derart großen Durchmesser hat, dass der Schneiddraht ungehindert in seiner axialen Richtung hindurch bewegt werden kann, und andererseits so klein ist, dass auf dem Schneiddraht haftende Gewebereste beim Hinein-schieben des Schneiddrahts in dieses Loch von der Oberfläche des Schneiddrahts abgestreift werden.

8. HF-chirurgisches Instrument nach einem der vorhergehenden Ansprüche,
**dadurch gekennzeichnet, dass**
eine elektrische Leitung zwischen dem Kabel (7) oder dem Anschluss für ein Kabel zum HF-chirurgischen Schneiddraht (5) innerhalb mindestens einer der Branchen (2a, 2b) oder außerhalb der Branchen geführt ist.
